# EUROPÄISCHE PATENTANMELDUNG

(11) **EP 2 881 729 A1**
(43) Veröffentlichungstag der Anmeldung: **10.06.2015**
(21) Anmeldenummer: 14189032.7
(22) Anmeldetag: 15.10.2014
(51) Int. Cl.: G01N 21/84, G01N 33/32, G01N 21/90

(54) **Messung des Aushärtegrades der Bedruckung oder Beschichtung eines Behälters**

(30) Priorität: 05.12.2013 DE 102013224949
(71) Anmelder: Krones AG, 93073 Neutraubling (DE)
(72) Erfinder: Sonnauer, Andreas, 93073 Neutraubling (DE); Lauterbach, Florian, 93073 Neutraubling (DE)
(74) Vertreter: Grünecker Patent- und Rechtsanwälte PartG mbB

(57) **Zusammenfassung**

Verfahren zum Messen des Aushärtegrades einer Bedruckung oder Beschichtung (10) eines Behälters (4) mit strahlungshärtender Farbe oder strahlungshärtendem Lack, gekennzeichnet dadurch, dass die Messung am Behälter (4) optisch erfolgt sowie eine Vorrichtung zur Durchführung eines solchen Verfahrens und computerlesbares Medium mit computerlesbaren Instruktionen zur Steuerung der Durchführung eines solchen Verfahrens.

## Beschreibung

Die Erfindung bezieht sich auf ein Verfahren zum Messen des Aushärtegrades einer Bedruckung oder Beschichtung eines Behälters mit strahlungshärtender Farbe oder strahlungshärtendem Lack, eine Vorrichtung zur Durchführung eines solchen Verfahrens und ein computerlesbares Medium mit computerlesbaren Instruktionen zur Steuerung der Durchführung eines solchen Verfahrens.

Strahlungshärtende Farben und Lacke, z.B. UV-härtende Farben und Lacke, werden häufig für Lackierung oder Beschriftung eingesetzt. Solche Farben und Lacke sind üblicherweise mit Photoinitiatoren versetzt, die bei Bestrahlung Radikale bilden und damit die Polymerisation, also die Härtung, starten, wodurch die Farben/Lacke schnell aushärten. Üblicherweise sind dabei die spektrale Empfindlichkeit der Photoinitiatoren und das Spektrum der Strahlungsquelle aufeinander abgestimmt.

Es gibt eine Vielzahl von Photoinitiatoren und Strahlungsquellen, z.B. UV-Quellen. Die Härtung einer Farbe/eines Lacks hängt von vielen Faktoren ab. Beispielsweise hängt sie von der Strahlungsdosis und der Spektralverteilung der Strahlungsquelle, von der Art und Menge der Photoinitiatoren, von Wärmeeintrag und Abfuhr, Schichtdicke, Anwesenheit von Radikalfängern wie Sauerstoff, eingesetzten Monomeren, funktionalen Vernetzungsstellen, Pigmentgehalt und weiteren Faktoren ab. In Systemen werden üblicherweise die eingesetzten Komponenten aufeinander abgestimmt, so dass sich die Abhängigkeit auf die Strahlungsdosis der eingesetzten Strahlungsquelle reduziert. Diese hängt wiederum von der Strahlungsintensität und der Verweildauer der Farbe/des Lacks im Intensitätsfeld der Strahlung ab, die beispielsweise mit der Produktionsgeschwindigkeit und/oder dem Abstand zur Strahlungsquelle korreliert sein kann.

Solche strahlungshärtenden Farben und Lacke werden auch zum Bedrucken oder Beschichten von Behältern eingesetzt. Wird nun bei solchen Behältern die Bedruckung oder Beschichtung nicht oder nur unvollständig gehärtet, beispielsweise aufgrund von Ausfall oder Schwankungen in der Intensität der Strahlungsquelle oder Änderungen der Produktionsgeschwindigkeit, kann die Bedruckung oder Beschichtung verschmieren. Auch kann es zu einer Migration der nicht gehärteten Farbe/des nicht gehärteten Lacks durch den Behälter in den Inhalt hinein kommen, so dass dieser durch die Farbe/den Lack verunreinigt wird.

Bis jetzt wurde die Aushärtung der Farbe/des Lacks bei Behältern nur stichprobenweise, indirekt und/oder unter Zerstörung des Behälters oder der Bedruckung oder Beschichtung überprüft. Üblich ist z. B. eine stichprobenartige Kontrolle der aufgebrachten UV-Dosis, beispielsweise über UV-Strips (z. B. Hönle UV-Scan mit tesa UV-Strips) mit anschließendem Abgleich zum erforderlichem Dosis-Messwert. Hierüber erfolgt dann indirekt eine Aussage zum Aushärtegrad.

Der Erfindung liegt die Aufgabe zugrunde, eine verbesserte Messung des Aushärtegrades einer Bedruckung oder Beschichtung eines Behälters zu ermöglichen.

Die Erfindung umfasst ein Verfahren zum Messen des Aushärtegrades einer Bedruckung oder Beschichtung nach Anspruch 1, eine Vorrichtung nach Anspruch 14 und ein computerlesbares Medium nach Anspruch 15. Vorteilhafte Ausführungsformen sind in den abhängigen Ansprüchen beschrieben.

Ein erfindungsgemäßes Verfahren zum Messen des Aushärtegrades einer Bedruckung oder Beschichtung eines Behälters mit einer strahlungshärtenden Farbe oder einem strahlungshärtenden Lack (im Folgenden auch: Farbe oder Lack) erfolgt optisch am Behälter. Im folgenden Text kann der Begriff Farbe oder Lack auch eine Kombination aus einer oder mehreren Farben und/oder einem oder mehreren Lacken umfassen. Der Begriff "Lack" kann hierbei auch farbige oder farblose Beschichtungen umfassen, z. B. Schichten aus Primer oder andere funktionale Schichten.

Üblicherweise ist die Farbe oder der Lack UV-härtend. Die Farbe oder der Lack können aber zusätzlich oder alternativ in anderen Spektralbereichen als dem UV härtend sein.

Behälter können in diesem Zusammenhang Glas, Kunststoff, Metall und/oder Karton umfassen. Insbesondere können es Getränkebehälter, z.B. Flaschen, aus Glas oder Kunststoff, wie beispielsweise PET, Dosen, z. B. aus Metall, oder Getränkebehälter aus Karton sein.

Eine Messung direkt am Behälter ist vorteilhaft, da dadurch Zwischenschritte vermieden werden, die oft zeitaufwendig sind und/oder die Messung ungenau machen. Zudem kann so in einigen Ausführungsformen eine 100 % Kontrolle, also eine Kontrolle jedes einzelnen Behälters an mindestens einer Stelle, erfolgen und/oder Produkte nachverfolgt werden.

Üblicherweise erfolgt die optische Messung in einem erfindungsgemäßen Verfahren, indem die Bedruckung oder Beschichtung mit Strahlung aus einer Strahlungsquelle, beispielsweise Licht aus einem Spektralbereich zwischen UV und Ferninfrarot, z.B. aus dem Mittleren Infrarotbereich (MIR), dem Nahen Infrarotbereich (NIR) oder sichtbarem Bereich (Vis) oder UV Bereich (UV), bestrahlt wird. Anschließend kann dann die Wechselwirkung gemessen werden, also beispielsweise die reflektierte Strahlung detektiert werden und daraus der Aushärtegrad bestimmt, also gemessen werden. Die Bestrahlung kann dabei z. B. unter einem flachen Winkel erfolgen. Eine Detektion der reflektierenden Strahlung kann dann z. B. um einen Winkel von zwischen 45° und 135°, z. B. zwischen 75° und 105°, beispielsweise ungefähr 90° gegenüber der einfallenden Strahlung versetzt erfolgen. In anderen Ausführungsformen kann die transmittierte Strahlung detektiert und daraus der Aushärtegrad bestimmt (gemessen) werden. Die Messung kann z. B. bezüglich einer Referenz ausgewertet werde.

Je nach vorhandenen Molekülen in der Bedruckung/Beschichtung zeigt die ungehärtete Farbe oder der ungehärtete Lack eine andere reflektierte (transmittierte) Strahlung bzw. Spektrum als die gehärtete Farbe oder der gehärtete Lack. Beispielsweise kann das Licht mit einer für den Aushärtegrad typischen veränderten Spektralverteilung, beispielsweise Abschwächung bestimmter Spektralbereiche, reflektiert (transmittiert) werden.

Ist beispielsweise bekannt, dass ein Photoinitiator vor seinem Zerfall ein charakteristisches Signal in einem bestimmten Spektralbereich im reflektierten (transmittierten) Licht hervorruft, bei der Strahlungshärtung aber zerfällt, sollte in der gehärteten Farbe oder Lack dieses charakteristische Signal geringer als in der ungehärteten Farbe oder dem ungehärteten Lack auftreten. Eine Analyse kann sich zusätzlich oder alternativ auch auf die Monomere und/oder weitere Bestandteile wie bestimmte Molekülgruppen oder Bindungsgruppen stützen, die das Spektrum der vom Behälter reflektierten Strahlung beeinflussen und im ungehärteten und gehärteten Zustand verschiedene charakteristische Signale in der reflektierten (transmittierten) Strahlung zeigen, z.B. weil im ungehärteten Zustand das reflektierte (transmittierte) Spektrum Signale zeigt, die einer bestimmten Molekülgruppe zuordenbar sind, die beim Härten zerfällt. Dadurch ist nach dem Härten das charakteristische Signal üblicherweise geringer ausgeprägt. Zugleich können neue charakteristische Signale im Spektrum auftauchen, die von der Wechselwirkung der Strahlung mit den Zerfalls- und/oder Polymerisationsprodukten hervorgerufen werden. Im Wesentlichen werden durch die Wechselwirkung mit der elektromagnetischen Strahlung üblicherweise elektronische oder Schwingungszustände angeregt.

In einem Verfahren zum Messen des Aushärtegrades kann eine Eichreihe mit Messungen der verwendeten ungehärteten, teilweise gehärteten und vollständig gehärteten Farbe oder des verwendeten ungehärteten, teilweise gehärteten und vollständig gehärteten Lacks für die Kalibrierung verwendet werden.

Als Strahlungsquellen für die optische Messung können kohärente Lichtquellen, wie z.B. Laser, und nicht-kohärente Lichtquellen, wie z.B. LED-Lampen, Hg-Lampen oder normale Glühlampen, verwendet werden.

Die Messung kann berührungslos erfolgen. Dies bedeutet, dass während der Messung der Behälter von den Messinstrumenten nicht berührt wird. Dies kann vorteilhaft sein, da die Messung dadurch schneller durchführbar sein kann und/oder mechanische Beschädigungen ausgeschlossen werden können.

In anderen Ausführungsformen kann die Messung mit einer Berührung des Behälters durch eines oder mehr Messinstrumente erfolgen, beispielsweise kann ein Lichtwellenleiter oder ähnliches den Behälter berühren.

Die Messung kann zerstörungsfrei erfolgen. Insbesondere wird z.B. die Bedruckung oder Beschichtung und der Behälter durch die zur Messung verwendete Strahlung nicht beschädigt. Dadurch kann anstatt einer stichprobenartigen in einigen Ausführungsformen eine 100-%-Prüfung jedes einzelnen Behälters erfolgen.

Ein oben beschriebenes Verfahren kann so erfolgen, dass das Ergebnis der Messung einem Bedruck- oder Beschichtvorgang des Behälters zuordenbar ist. Beispielsweise können in einem Verfahren Behälter vermessen werden, die in einer bestimmten Reihenfolge angeliefert werden, die eine Zuordnung zu einem Bedruck- oder Beschichtvorgang erlaubt. So kann das Messen des Aushärtegrades so erfolgen, dass für jeden gemessenen Behälter bekannt ist oder feststellbar ist, wann dieser beschichtet oder bedruckt wurde. Dies kann vorteilhaft sein, da dadurch beim Auftreten von Fehlern, beispielsweise eine Schwankung in der Intensität der Strahlungsquelle, der Produktionsgeschwindigkeit und/oder dem Auftrag der Bedruckung/Beschichtung, einfacher auf die Fehlerursache oder den Zeitpunkt des Fehlereintrittes geschlossen werden kann.

Beispielsweise kann ein Behälter zuerst bedruckt oder beschichtet, dann die Bedruckung oder Beschichtung mit Strahlung aus einer Strahlungsquelle, beispielsweise einer UV-Quelle, gehärtet und anschließend eine Messung des Aushärtegrades der Bedruckung oder Beschichtung des Behälters durchgeführt werden. Das Bedrucken oder Beschichten und Aushärten kann in einem erfindungsgemäßen Verfahren umfasst sein. In anderen Ausführungsformen von erfindungsgemäßen Verfahren ist es nicht umfasst.

In einigen Ausführungsformen eines erfindungsgemäßen Verfahrens können mehrere Behälter vermessen werden. Beispielsweise können Behälter bedruckt oder beschichtet und dann die Bedruckung oder Beschichtung mit Strahlung gehärtet werden. Der Aushärtegrad kann für jeden bedruckten oder beschichteten Behälter oder in regelmäßigen Abständen, beispielsweise für jeden zehnten bedruckten oder beschichteten Behälter, gemessen werden.

In einem Verfahren zum Messen des Aushärtegrades kann der Schritt umfasst sein, Behälter, bei denen der Aushärtegrad der Bedruckung oder Beschichtung nicht ausreichend ist, auszusortieren.

Alternativ oder zusätzlich kann das Ergebnis der Messung zur Steuerung der Parameter beim Bedrucken oder Beschichten und/oder beim Aushärten der Farbe oder des Lacks für folgende Behälter verwendet werden. Beispielsweise kann beim Aushärten der Farbe oder des Lacks die Intensität der Strahlungsquelle und/oder die Schichtdicke der Bedruckung oder Beschichtung und/oder die Verweildauer des bedruckten oder beschichteten Behälters im Strahlungsfeld der Strahlungsquelle zum Härten der Farbe oder des Lacks, beispielsweise über Regelung der Produktionsgeschwindigkeit und/oder des Abstands zwischen Behälter und Strahlungsquelle, angepasst werden. Wird also z.B. bei einem Behälter ein zu geringer Aushärtegrad der Bedruckung oder Beschichtung gemessen, können die zuvor beschriebenen Parameter so angepasst werden, dass die folgenden Behälter einen hinreichenden Aushärtegrad zeigen, beispielsweise die Schichtdicke der Bedruckung/Beschichtung gesenkt, die Intensität der Strahlungsquelle erhöht und/oder die Verweildauer im Intensitätsfeld der Strahlungsquelle zur Härtung erhöht werden.

In einem Verfahren kann dadurch ein Regelkreis zur Optimierung der Parameter implementiert werden. Der Aushärtegrad der Bedruckung oder Beschichtung kann dabei in regelmäßigen Abständen, beispielsweise für jeden zehnten Behälter, oder für jeden Behälter gemessen werden.

Die Frequenz, mit der die Messung des Aushärtegrades der Bedruckung oder Beschichtung eines Behälters erfolgt, kann mehr als 0,02 Hz betragen. Beispielsweise kann sie mehr als 0,1 Hz, beispielsweise mehr als 1 Hz, z.B. mehr als 10 Hz, beispielsweise mehr als 20 Hz, z. B. mehr als 30 Hz, betragen.

Mit einem erfindungsgemäßen Verfahren können z. B. für bis zu 120 000 Behälter pro Stunde, z. B. für bis zu 3 600 Behälter pro Stunde, z. B. für bis zu 3 000 Behälter pro Stunde der Aushärtegrad der Bedruckung oder Beschichtung gemessen werden.

Bei einem Verfahren zum Messen des Aushärtegrades kann die Messung in einer (horizontalen) Zeile der Bedruckung oder Beschichtung erfolgen. Insbesondere kann der Aushärtegrad an einem Punkt der Zeile, in einem Teilbereich der Zeile oder über den ganzen Behälterumfang hinweg gemessen werden. Dabei kann ein durchschnittlicher Aushärtegrad für den gesamten gemessenen Bereich gebildet werden oder für mehrere Teilbereiche jeweils einzelne Aushärtegrade bestimmt werden.

In anderen Ausführungsformen kann die Messung einen größeren Bereich in vertikaler Richtung umfassen und/oder flächig erfolgen. Beispielsweise kann die Messung über mehrere Zeilen hinweg erfolgen. Hierbei kann für jede der gemessenen Zeilen der Aushärtegrad an einem Punkt, in einem Bereich oder über den ganzen Behälterumfang hinweg gemessen werde. Es kann ein durchschnittlicher Aushärtegrad für den gesamten gemessenen Bereich gebildet werden oder für mehrere Teilbereiche jeweils einzelne Aushärtegrade bestimmt werden. Eine Messung über einen größeren Bereich in vertikale Richtung oder eine flächige Messung können vorteilhaft sein, da sie schwankende Aushärtgrade, beispielsweise aufgrund von verschiedenen Schichtdicken der Bedruckung/Beschichtung erkennen können. In anderen Ausführungsformen ist die Messung in einer (horizontalen) Zeile der Bedruckung oder Beschichtung ausreichend.

In einem erfindungsgemäßen Verfahren kann die Auswertung automatisch, beispielsweise mittels einer Auswertsoftware, erfolgen. Damit kann beispielsweise einem Benutzer, z.B. über einen Bildschirm, als Ergebnis die Information ausgegeben werden, ob der Aushärtegrad ausreichend und/oder konstant über mehrere Behälter ist oder nicht. Dies kann vorteilhaft sein, da dadurch die Komplexität für den Benutzer reduziert wird. Die automatische Auswertung kann z.B. nach vom Benutzer vorgegebenen Kriterien, wie Vergleich von integrierten Spektren von Frequenzbereichen untereinander und/oder mit Referenzen und/oder aufgrund von Eichreihen erfolgen. In einigen Ausführungsformen kann die Auswertung zusätzlich oder alternativ manuell erfolgen. Dies kann eine differenziertere Auswertung nach Angaben des Benutzers ermöglichen.

Die Messung kann in einem erfindungsgemäßen Verfahren in Vergleich zu einer Referenz (z.B. einer Eichreihe mit einem Behälter mit vollständig gehärteter und einem Behälter mit vollständig ungehärteter und optional Behältern mit teilweise gehärteter Bedruckung oder Beschichtung) gesetzt werden und daraus ein Ergebnis abgeleitet werden. In anderen Ausführungsformen erfolgt die Angabe eines Ergebnisses ohne Referenz, z.B. aufgrund eines Vergleichs der Integrale verschiedener Frequenzbereiche zueinander.

In einem erfindungsgemäßen Verfahren kann die Messung eine quantitative Aussage über den Aushärtegrad erlauben. Beispielsweise kann durch Vergleich von bestimmten Frequenzbereichen des oder der Reflexionsspektren (oder Transmissionsspektren) eine Aussage über den Anteil von nicht (vollständig) gehärteter Farbe oder Lack getroffen werden und damit eine quantitative Aussage über den Aushärtegrad getroffen werden.

Ein erfindungsgemäßes Verfahren kann einen Trainingsschritt umfassen, in dem die Messwerte oder Messergebnisse bzw. die daraus resultierende Interpretation auf die zu vermessende Behältersorte und/oder die verwendete Farbe oder den verwendeten Lack und/oder Schichtdicke der Bedruckung oder Beschichtung kalibriert werden. Dies kann z.B. durch Eichreihen erfolgen, wie sie zuvor beschrieben wurden. Messwerte oder Messergebnisse können dann z.B. mit Referenzen aus Eichreihen verglichen werden und der Aushärtegrad daraus bestimmt werden, so dass eine korrekte Interpretation und Ergebnisausgabe erfolgen kann.

Ein erfindungsgemäßes Verfahren kann NIR und MIR-Spektroskopie, Aufnehmen eines oder mehrerer Bilder mit einer oder mehr NIR-Kameras, die z.B. flächige Aufnahmen machen können und damit z.B. visuell die flächige Verteilung des Aushärtegrades darstellen können, UV-Vis-Spektroskopie, die z.B. Anregungen in konjugierten oder aromatischen Systemen, z.B. Monomeren und/oder Photoinitiatoren, erfassen kann, Fluoreszenzspektroskopie, die Fluoreszenzen oder Phosphoreszenzen z.B. von Monomeren und/oder Photoinitiatoren erfassen kann und/oder Ramanspektroskopie, die z.B. bestimmte Molekülschwingungen erfassen kann, umfassen.

In einem Verfahren zum Messen des Aushärtegrades einer Bedruckung oder Beschichtung kann der Behälter (4) während des Messvorgangs bewegt werden. Beispielsweise kann der Behälter linear am Ort, wo die Messung durchgeführt wird, vorbeitransportiert werden oder getaktet am Ort, wo die Messung durchgeführt wird, vorbeigeführt werden und dabei beispielsweise einmal in horizontaler Richtung um sich selbst, beispielsweise um eine halbe oder ganze Umdrehung, gedreht werden. Somit kann der Auftreffpunkt der zur Messung verwendeten Strahlung einen horizontalen Bereich, z.B. einen halben oder ganzen Umfang des Behälters horizontal abtasten und die Messung diesen Bereich erfassen.

Dabei kann der Bewegung zusätzlich noch eine weitere, beispielsweise lineare, Bewegung überlagert werden. So kann beispielsweise für Behälter mit ovaler Grundfläche der Abstand zur Lichtquelle während einer Drehung etwa konstant gehalten werden.

Zusätzlich oder alternativ kann/können die Auftreffposition(en) der zur Messung verwendeten Strahlung aus einer Strahlungsquelle während einer Messung in vertikaler und/oder horizontaler Richtung verändert werden. Beispielsweise kann, wenn die Strahlung über einen oder mehrere Lichtwellenleiter zur Bedruckung oder Beschichtung geführt wird, der oder die Lichtwellenleiter vertikal und/oder horizontal bewegt werden, z.B. in einer Pendelbewegung, und/oder der Fokuspunkt/die Fokuspunkte der Strahlung vertikal und/oder horizontal bewegt werden.

In einigen Ausführungsformen ist die Auftreffposition der Strahlung aufgeweitet, beispielsweise linienförmig (in vertikale Richtung) oder flächig.

Die Detektion der reflektierten (transmittierten) Strahlung bei der optischen Messung kann über einen oder mehrere Kanäle oder flächig erfolgen. Eine Aufnahme der Spektren über einen Kanal erlaubt ein Ergebnis einfach und schnell festzustellen. Eine Auswertung über mehrere Kanäle oder flächig erlaubt, ein Profil der Aushärtegrade für bestimmte Punkte oder Bereiche oder Flächen zu erstellen.

Die Erfindung umfasst des Weiteren eine Vorrichtung, die Mittel zum Durchführen eines zuvor beschriebenen Verfahrens umfasst. Die Vorrichtung umfasst eine, zwei oder mehr Strahlungsquellen für die zur Messung verwendete Strahlung und einen, zwei, drei oder mehr Detektoren für die von der Bedruckung oder Beschichtung reflektierte (transmittierte) Strahlung. Sie kann auch Mittel zur Durchführung weiterer Verfahrensschritte, beispielsweise zum Bedrucken oder Beschichten von Behältern mit strahlungshärtender Farbe oder strahlungshärtendem Lack und/oder Aushärten der Farbe oder des Lacks umfassen.

Die Strahlungsquelle und der Detektor zum Messen des Aushärtegrades können in einem eigenen Bauteil umfasst sein, das an Druckvorrichtungen hinten angebaut oder hinter der Druckvorrichtung installiert werden kann. In anderen Ausführungsformen sind Strahlungsquelle und Detektor zum Messen des Aushärtegrades in der Druckvorrichtung umfasst und dort nach der Strahlungsquelle zum Härten der Farbe oder des Lacks angeordnet.

Die Erfindung umfasst außerdem ein computerlesbares Medium, das computeriesbare Instruktionen umfasst, die, wenn sie von einem Computer ausgeführt werden, die Durchführung eines zuvor beschriebenen Verfahrens und/oder eine zuvor beschriebene Vorrichtung steuern können.

Vorteilhafte Ausführungsformen werden anhand der Figuren beschrieben. Hierbei zeigt
- Fig. 1: schematisch einen Teil einer beispielhaften Vorrichtung;
- Fig. 2: ein Detail einer möglichen Anordnung von Behälter und Strahlungsquelle in einer beispielhaften Vorrichtung;
- Fig. 3: schematische Ausführungsformen von beispielhaften Vorrichtungen.

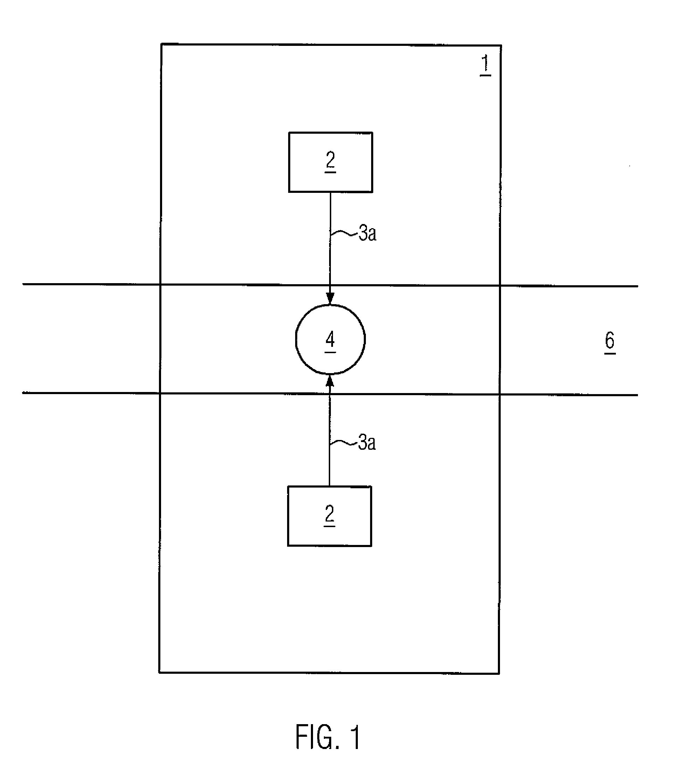

Fig. 1 zeigt schematisch und nicht maßstabsgetreu einen Teil einer beispielhaften Ausführungsform einer erfindungsgemäßen Vorrichtung. Vorrichtung 1 als Teil einer beispielhaften Vorrichtung kann in einer erfindungsgemäßen Vorrichtung umfasst sein. Insbesondere umfasst die in diesem Beispiel gezeigte Vorrichtung 1 zwei Strahlungsquellen 2. In einem erfindungsgemäßen Verfahren können die Bedruckung oder Beschichtung von zwei gegenüberliegenden Seiten des Behälters 4 gleichzeitig vermessen werden, z.B. wenn es in einer Vorrichtung, die Vorrichtung 1 umfasst, durchgeführt wird.

In anderen, hier nicht gezeigten Ausführungsformen, umfasst die Vorrichtung 1 nur eine Strahlungsquelle, deren Strahlung, optional von genau einer Seite, auf den Behälter geleitet wird, z. B. über einen Lichtwellenleiter oder Fokussierung, oder deren Strahlung optional über verschiedene Lichtwellenleiter oder Fokussierung von zwei oder mehr Seiten auf den Behälter geleitet wird, oder mehr als 2 Strahlungsquellen.

Eingezeichnet ist ebenfalls die von der Strahlungsquelle auf die Bedruckung oder Beschichtung fallende Strahlung 3a.

Behälter 4 ist hierbei in der Messposition, in der der Aushärtegrad der Bedruckung oder Beschichtung gemessen wird, auf einer Behälterbahn 6 gezeigt, auf der Behälter 4 durch die Vorrichtung 1 transportiert werden können. Sie kann einen Förderer oder eine andere Transportvorrichtung für Behälter, beispielsweise mit einzelnen Drehtellern für Behälter, umfassen oder als solche ausgebildet sein. In anderen Ausführungsformen kann der Behälter auf andere Weise in die Vorrichtung 1 und aus ihr heraus transportiert werden.

In einigen Ausführungsformen bewegt sich der Behälter 4, während die Messung des Aushärtegrades erfolgt. In diesem Fall gibt es keine Messposition, sondern einen flächigen Messbereich, in dem die Messung erfolgt (hier nicht gezeigt).

Der eine, zwei oder die mehr Detektoren zur Detektion der reflektierten (oder transmittierten) Strahlung sind in Figur 1 nicht gezeigt.

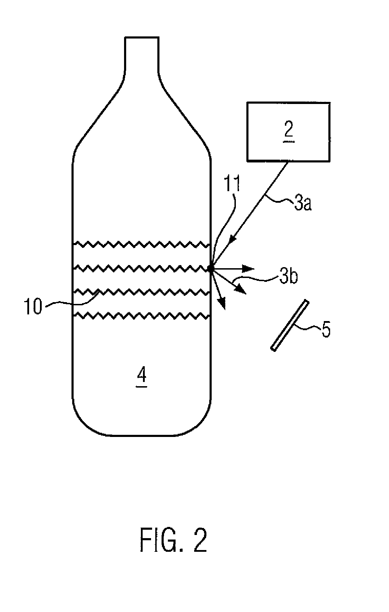

Figur 2 zeigt eine schematische Darstellung eines Details einer möglichen Anordnung von Behälter und Strahlungsquelle in einer Ausführungsform einer erfindungsgemäßen Vorrichtung. So eine Anordnung kann beispielsweise in einem Vorrichtungsteil wie in Figur 1 gezeigt umfasst sein. Hierbei befindet sich der Behälter 4 am Ort, wo die Messung durchgeführt wird (in der Messposition bzw. im Messbereich). Beispielhaft ist in Fig. 2 eine Strahlungsquelle eingezeichnet. In anderen Ausführungsformen können zwei, drei oder mehr Strahlungsquellen vorhanden sein und/oder die Strahlung aus einer oder mehr Strahlungsquellen über verschiedene Lichtwellenleiter oder Fokussierung von zwei oder mehr Seiten auf den Behälter geleitet werden (nicht gezeigt).

Die auf die Bedruckung oder Beschichtung 10 fallende Strahlung 3a trifft auf der Bedruckung oder Beschichtung 10 an Auftreffposition 11 auf (die Auftreffposition kann punktförmig sein, wie hier gezeigt oder in vertikaler und/oder horizontaler Richtung jeweils einen größeren Bereich als einen Punkt, beispielsweise eine Linie oder eine Fläche umfassen). Die auf die Bedruckung oder Beschichtung 10 fallende Strahlung wird in dem gezeigten Beispiel als Strahlung 3b reflektiert. Die reflektierte Strahlung 3b wird von einem, zwei oder mehr Detektoren 5 detektiert (beispielhaft ist ein Detektor gezeichnet) und kann anschließend ausgewertet werden. Daraus kann der Aushärtegrad der Bedruckung oder Beschichtung für eine, zwei, drei oder mehr Punkte oder Bereiche gemessen werden.

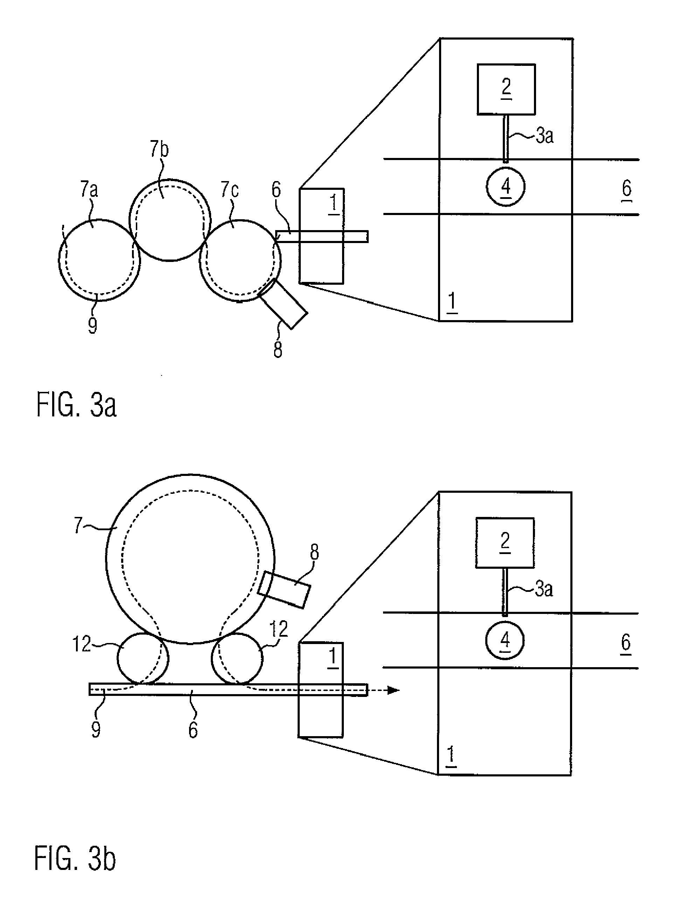

In Figur 3a und Figur 3b werden schematisch Ausführungsformen von erfindungsgemäßen Vorrichtungen gezeichnet. Die Darstellung ist dabei weder vollständig noch maßstabgetreu.

Die Behälter 4 werden hierbei jeweils in einem Behälterstrom 9 zuerst durch eine Druckvorrichtung (7, 7a, 7b, 7c) geführt, die in einer erfindungsgemäßen Vorrichtung umfasst sein können, wo die Farbe oder der Lack für die Bedruckung oder Beschichtung aufgebracht wird. Anschließend wird die Farbe oder der Lack mit Strahlung aus Strahlungsquelle 8 gehärtet.

Anschließend werden in den hier gezeigten Beispielen die Behälter 4 auf Behälterbahn 6 durch eine Vorrichtung 1 transportiert, wo mittels Strahlungsquelle 2 und Strahlung 3a der Aushärtegrad der Bedruckung oder Beschichtung des Behälters 4 gemessen wird. In den in Figur 3 gezeigten Beispielen kann jeweils die Reihenfolge der Behälter erhalten bleiben, so dass nach dem Messen das Messergebnis einem Druckvorgang zuordenbar ist. Auch kann in den in Figur 3 gezeigten Beispielen der Aushärtegrad für jeden einzelnen Behälter bestimmt werden, der zuvor bedruckt oder beschichtet wurde.

In anderen Ausführungsformen der Vorrichtung ist die Vorrichtung 1 auf der Druckvorrichtung 7, 7c aufgebaut oder in die Druckvorrichtung 7, 7c integriert, und zwar nach der Strahlungsquelle 8 zur die Härtung der Farbe oder des Lacks (nicht gezeigt). In einigen Ausführungsformen kann die Härtung der Farbe oder des Lacks durch die gleiche Strahlungsquelle wie das Messen des Aushärtegrades erfolgen (nicht gezeigt), beispielsweise durch Detektion der reflektierten (transmittierten) spektralen UV-Vis-Anteile einer UV-Strahlungsquelle.

In Figur 3a erfolgt in jeder Druckvorrichtung 7a, 7b und 7c der Druck mit einer Farbe oder einem Lack einer Farbe. Anschließend werden die (in diesem Beispiel drei) aufgetragenen Farben (oder Lacke) gemeinsam durch Strahlung aus Strahlungsquelle 8 gehärtet.

In Figur 3b erfolgt der Druck mit allen Farben oder Lacken in Vorrichtung 7, anschließend werden die Farben oder Lacke durch Strahlung aus Strahlungsquelle 8 gehärtet.

In dem in Figur 3b gezeigten Beispiel erfolgt die Zuleitung des Behälterstromes 9 in Druckvorrichtung 7 von der Behälterbahn 6 über eine Transfervorrichtung 12, die in diesem Beispiel beispielhaft als Transferkarussell ausgebildet ist. Die Ableitung der Behälter aus der Druckvorrichtung erfolgt ebenfalls über eine Transfervorrichtung 12, die in diesem Beispiel beispielhaft als Transferkarussell ausgebildet ist.

Anschließend werden die Behälter 4 im Behälterstrom 9 durch Vorrichtung 1 transportiert, wo der Aushärtegrad der Bedruckung oder Beschichtung bestimmt wird.

## Patentansprüche

1. Verfahren zum Messen des Aushärtegrades einer Bedruckung oder Beschichtung (10) eines Behälters (4) mit strahlungshärtender Farbe oder strahlungshärtendem Lack, **gekennzeichnet dadurch, dass** die Messung am Behälter (4) optisch erfolgt.

2. Verfahren zum Messen des Aushärtegrades einer Bedruckung oder Beschichtung (10) eines Behälters (4) nach Anspruch 1, wobei die Messung berührungslos erfolgt.

3. Verfahren zum Messen des Aushärtegrades einer Bedruckung oder Beschichtung (10) eines Behälters (4) nach Anspruch 1 oder Anspruch 2, wobei die Messung zerstörungsfrei erfolgt.

4. Verfahren zum Messen des Aushärtegrades einer Bedruckung oder Beschichtung (10) eines Behälters (4) nach einem der Ansprüche 1 bis 3, wobei das Ergebnis dem Bedruck- oder Beschichtvorgang des Behälters diskret zuordenbar ist.

5. Verfahren zum Messen des Aushärtegrades einer Bedruckung oder Beschichtung (10) eines Behälters (4) nach einem der Ansprüche 1 bis 4, wobei ein Behälter (4), bei dem der Aushärtegrad der Bedruckung oder Beschichtung (10) nicht ausreichend ist, aussortiert wird.

6. Verfahren zum Messen des Aushärtegrades einer Bedruckung oder Beschichtung (10) eines Behälters (4) nach einem der Ansprüche 1 bis 5, wobei das Ergebnis der Messung zur Steuerung der Parameter beim Bedrucken oder Beschichten und/oder Aushärten der strahlungshärtenden Farbe oder des strahlungshärtenden Lacks für folgende Behälter (4) verwendet wird.

7. Verfahren zum Messen des Aushärtegrades einer Bedruckung oder Beschichtung (10) eines Behälters (4) nach einem der Ansprüche 1 bis 6, wobei die Frequenz, mit der eine Messung des Aushärtegrades wiederholt wird, mehr als 0,02 Hz beträgt.

8. Verfahren zum Messen des Aushärtegrades einer Bedruckung oder Beschichtung (10) eines Behälters (4) nach einem der Ansprüche 1 bis 7, wobei die Messung einen Punkt, eine Zeile, einen größeren Zeilenbereich oder einen flächigen Bereich der Bedruckung oder Beschichtung (10) erfasst.

9. Verfahren zum Messen des Aushärtegrades einer Bedruckung oder Beschichtung (10) eines Behälters (4) nach einem der Ansprüche 1 bis 8, wobei die Auswertung automatisch erfolgt.

10. Verfahren zum Messen des Aushärtegrades einer Bedruckung oder Beschichtung (10) eines Behälters (4) nach einem der Ansprüche 1 bis 9, wobei die Messung eine quantitative Aussage über den Aushärtegrad erlaubt.

11. Verfahren zum Messen des Aushärtegrades einer Bedruckung oder Beschichtung (10) eines Behälters (4) nach einem der Ansprüche 1 bis 10, wobei das Verfahren einen Trainingsschritt umfasst, bei dem die Messwerte auf die zu messende Behältersorte und/oder eine oder mehr strahlungshärtende Farben und/oder einen oder mehr strahlungshärtende Lacke und/oder Dicke der Bedruckung oder Beschichtung (10) kalibriert werden.

12. Verfahren zum Messen des Aushärtegrades einer Bedruckung oder Beschichtung (10) eines Behälters (4) nach einem der Ansprüche 1 bis 11, wobei bei der Messung eine oder mehr der folgenden Techniken oder Mittel Anwendung findet:
NIR und/oder MIR Spektroskopie;
NIR Kameras;
UV-Vis Spektroskopie;
Fluoreszenzspektroskopie;
Ramanspektroskopie.

13. Verfahren zum Messen des Aushärtegrades einer Bedruckung oder Beschichtung (10) eines Behälters (4) nach einem der Ansprüche 1 bis 12, wobei der Behälter (4) während des Messvorgangs bewegt wird und/oder die Auftreffposition (11) der zur Messung verwendeten Strahlung (3) aus einer Strahlungsquelle (2) während einer Messung in vertikale und/oder horizontale Richtung verändert wird.

14. Vorrichtung, die Mittel zum Durchführen eines Verfahrens nach einem der Ansprüche 1-13 umfasst mit einer Strahlungsquelle (2) für die zur Messung verwendeten Strahlung (3a) und einem Detektor (5) für Strahlung (3b).

15. Computerlesbares Medium, das computerlesbare Instruktionen umfasst, die, wenn sie von einem Computer ausgeführt werden, die Durchführung eines Verfahrens nach einem der Ansprüche 1 - 13 und/oder eine Vorrichtung nach Anspruch 14 steuern können.
